# FASCICULE DE BREVET EUROPEEN

(11) **EP 2 863 968 B1**
(45) Date de publication et mention de la délivrance du brevet: **05.09.2018**
(21) Numéro de dépôt: 13744604.3
(22) Date de dépôt: 21.06.2013
(51) Int. Cl.: A61M 5/32, A61M 5/31

(54) **ENSEMBLE D'INJECTION**
EINSPRITZANORDNUNG
INJECTION ASSEMBLY

(30) Priorité: 22.06.2012 FR 1255945
(43) Date de publication de la demande: 29.04.2015
(73) Titulaire: Laboratoire Aguettant, 69007 Lyon (FR)
(72) Inventeur: HUET, Gildas, F-69001 Lyon (FR)
(74) Mandataire: Verriest, Philippe
(86) Numéro de dépôt international: PCT/FR2013/051455
(87) Numéro de publication internationale: WO 2013/190249

(56) Documents cités:
- EP-A1- 2 462 969
- WO-A1-02/09797
- WO-A1-2009/097634
- US-A- 5 393 301
- US-A- 5 807 374
- US-B1- 6 315 113

## Description

La présente invention concerne un ensemble d'injection comprenant notamment un dispositif d'injection et un dispositif de protection.

Un dispositif d'injection, tel qu'une seringue préremplie, comporte, de façon connue, un embout de raccordement de type Luer ou Luer-Lock destiné à coopérer avec un embout de forme complémentaire permettant de raccorder une aiguille d'injection ou une ligne de perfusion au dispositif d'injection. Un embout de raccordement de type Luer est généralement composé d'une partie de raccordement tubulaire tronconique destinée au passage d'un fluide, tandis qu'un embout de raccordement de type Luer Lock est généralement composé en outre d'un manchon de verrouillage filetée coaxial à la partie de raccordement et entourant celle-ci.

Afin de recouvrir et protéger la partie de raccordement de l'embout de raccordement, et éventuellement d'obturer ladite partie de raccordement, un bouchon de protection est généralement vissé ou emmanché sur l'embout de raccordement.

Ainsi, lorsqu'un utilisateur souhaite monter une aiguille d'injection sur le dispositif d'injection, par exemple en vue d'injecter le contenu du dispositif d'injection dans un conteneur dépourvu d'embout de raccordement complémentaire de celui du dispositif d'injection, il doit nécessairement retirer le bouchon de protection du dispositif d'injection, ouvrir un conditionnement dans lequel est disposé une aiguille d'injection, saisir l'aiguille d'injection et la monter sur l'embout de raccordement du dispositif d'injection.

Une telle manipulation engendre un nombre de gestes importants pour l'utilisateur et multiplie de ce fait les risques de contamination de l'embout de raccordement du dispositif d'injection et/ou de l'aiguille d'injection.

La présente invention vise à remédier à ces inconvénients.

Le problème technique à la base de l'invention consiste donc à fournir un ensemble d'injection qui soit de structure simple et économique, et qui permette de réaliser aisément le montage d'une aiguille d'injection sur un dispositif d'injection, tout en limitant les risques de contamination.

Le document Us 5 807 374 A décrit un ensemble d'injection comprenant une portion de protection et un élément d'injection amovible. A cet effet, la présente invention concerne un ensemble d'injection, comportant :
- un dispositif d'injection prérempli comprenant un embout de raccordement, l'embout de raccordement comportant une partie de raccordement tubulaire destinée au passage d'un fluide, et
- un dispositif de protection comprenant :
   - une portion de protection montée de manière amovible sur l'embout de raccordement du dispositif d'injection de manière à recouvrir et protéger au moins en partie ledit embout de raccordement,
   - une portion de support liée à la portion de protection, et
   - un élément d'injection monté de manière amovible sur la portion de support dans une position de stockage, l'élément d'injection comprenant une aiguille d'injection et un embout de connexion relié fluidiquement à l'aiguille d'injection, l'embout de connexion étant adapté pour être monté sur l'embout de raccordement du dispositif d'injection dans une position d'utilisation de l'élément d'injection, l'élément d'injection et la portion de support étant agencés de telle sorte que, en position de stockage de l'élément d'injection, l'embout de connexion est accessible pour coopérer avec l'embout de raccordement.

Le montage de l'élément d'injection directement sur le dispositif de protection permet à un utilisateur, lorsque le dispositif de protection est monté sur le dispositif d'injection, de réaliser le montage de l'aiguille d'injection sur ce dispositif d'injection tout simplement en retirant la portion de protection du dispositif d'injection de manière à libérer l'embout de raccordement de ce dernier, et en faisant coopérer l'embout de connexion de l'élément d'injection avec l'embout de raccordement du dispositif d'injection.

Ainsi, l'ensemble d'injection selon l'invention permet un montage de l'aiguille d'injection sur le dispositif d'injection sans changer de main et sans avoir besoin d'ouvrir un autre conditionnement. Il en résulte un montage aisé de l'aiguille d'injection et une importante diminution des risques de contamination de l'embout de raccordement, et du fluide s'écoulant à travers ce dernier.

En outre, un tel montage de l'élément d'injection sur le dispositif de protection permet de conserver l'aiguille d'injection dans une position prête à l'emploi, tout en la maintenant éloignée du fluide contenu dans le dispositif d'injection. Ces dispositions permettent d'éviter tout problème d'incompatibilité entre l'aiguille d'injection et le fluide contenu dans le dispositif d'injection durant le stockage de ce dernier.

Selon un mode de réalisation de l'invention, le dispositif d'injection contient un liquide à usage médical.

Selon un mode de réalisation de l'invention, le dispositif d'injection peut être une seringue, une poche, une tube souple, un flacon ou une ampoule souple, ou encore une fiole souple.

Selon un mode de réalisation de l'invention, la portion de support est agencée pour recouvrir au moins en partie l'aiguille d'injection et au moins en partie l'embout de connexion. De préférence, la portion de support est agencée pour recouvrir au moins en partie l'aiguille d'injection de manière à empêcher tout contact entre l'extrémité libre de l'aiguille d'injection et les doigts d'un utilisateur.

Avantageusement, la portion de support comporte un logement de stockage dans lequel est monté de manière amovible l'élément d'injection.

Selon une caractéristique de l'invention, la portion de support comporte au moins une lumière de passage débouchant dans le logement de stockage et destinée au passage d'un fluide de stérilisation. Selon un mode de réalisation de l'invention, la portion de support comporte par exemple au moins une lumière de passage débouchant dans le logement de stockage au niveau de l'aiguille d'injection et/ou au moins une lumière de passage débouchant dans le logement de stockage au niveau de l'embout de connexion.

De préférence, la portion de support comporte une ouverture de passage débouchant dans le logement de stockage, l'ouverture de passage étant agencée pour permettre le retrait de l'élément d'injection hors du logement de stockage.

Le logement de stockage comporte par exemple une première partie dans laquelle s'étend l'aiguille d'injection, et une deuxième partie prolongeant la première partie et dans laquelle s'étend l'embout de connexion, l'ouverture de passage débouchant dans la deuxième partie du logement de stockage.

Selon un mode de réalisation de l'invention, l'embout de connexion est tubulaire et comporte une première extrémité ouverte reliée fluidiquement à l'aiguille d'injection, et une deuxième extrémité ouverte opposée à la première extrémité ouverte. Selon ce mode de réalisation, l'ouverture de passage est avantageusement située en regard de la deuxième extrémité ouverte de l'embout de connexion.

De façon avantageuse, la portion de support s'étend au-delà de la deuxième extrémité ouverte de l'embout de connexion.

Selon un mode de réalisation de l'invention, l'élément d'injection est relié à la portion de support par l'intermédiaire d'au moins une zone ruptible.

Selon un autre mode de réalisation de l'invention, l'élément d'injection coopère par friction avec au moins une portion de paroi délimitant le logement de stockage.

Selon un mode de réalisation de l'invention, le dispositif de protection comporte un corps monobloc comprenant les portions de protection et de support. Selon un mode de réalisation de l'invention, le corps monobloc est souple, et est par exemple réalisé en élastomère.

Selon un mode de réalisation de l'invention, l'élément d'injection est monobloc, et peut être par exemple métallique ou être réalisé en matière plastique.

Selon un mode de réalisation de l'invention, le dispositif de protection comprend un capuchon comportant la portion de protection, et un organe de support rapporté sur le capuchon et comportant la portion de support. Le capuchon et l'organe de support sont par exemple réalisés en matière plastique.

L'élément d'injection et la portion de support sont par exemple agencés de telle sorte que, en position de stockage de l'élément d'injection, l'aiguille d'injection s'étend sensiblement parallèlement à la direction générale d'extension du dispositif de protection.

Selon un mode de réalisation de l'invention, la portion de protection comporte au moins un orifice de passage destiné au passage d'un fluide de stérilisation.

Selon un mode de réalisation de l'invention, l'ensemble d'injection comprend des moyens d'obturation agencés pour obturer l'extrémité libre de la partie de raccordement de l'embout de raccordement.

Selon un mode de réalisation de l'invention, la portion de protection comprend les moyens d'obturation ou un logement d'accouplement agencé pour loger au moins en partie les moyens d'obturation.

Selon un mode de réalisation de l'invention, les moyens d'obturation comportent une paroi d'obturation prévue sur la portion de protection et agencée pour obturer l'extrémité libre de la partie de raccordement de l'embout de raccordement.

Selon un autre mode de réalisation de l'invention, les moyens d'obturation comportent un obturateur relié par une zone sécable à l'extrémité libre de la partie de raccordement de l'embout de raccordement. Avantageusement, le dispositif de protection comprend une portion d'accouplement agencée pour coopérer avec l'obturateur de sorte que la rotation de la portion d'accouplement autour d'un axe de rotation parallèle à la direction d'extension de la partie de raccordement entraîne en rotation l'obturateur de manière à provoquer la rupture de la zone sécable.

De préférence, la portion d'accouplement comporte un logement d'accouplement dans lequel est logé au moins en partie l'obturateur.

Selon un mode de réalisation de l'invention, l'embout de raccordement du dispositif d'injection est de type Luer ou Luer Lock. Selon un mode de réalisation de l'invention, l'embout de raccordement comporte un manchon de verrouillage fileté entourant la partie de raccordement.

Selon un mode de réalisation de l'invention, la portion de support et l'embout de connexion sont agencés de manière à délimiter un espace de passage adapté au passage du manchon de verrouillage de l'embout de raccordement lors du montage de l'élément d'injection sur l'embout de raccordement.

Selon un mode de réalisation de l'invention, la portion de protection comporte une jupe agencée pour être engagée sur la paroi externe du manchon de verrouillage de l'embout de raccordement. De préférence, l'orifice de passage est ménagé sur la jupe de la portion de protection. Avantageusement, la jupe et le manchon de verrouillage de l'embout de raccordement délimite une chambre interne dans laquelle débouche l'orifice de passage.

Avantageusement, le logement d'accouplement de la portion d'accouplement débouche dans le volume interne délimité par la jupe de la portion de protection.

Selon un autre mode de réalisation de l'invention, la portion de protection comporte un manchon de montage présentant une surface extérieure et une surface intérieure, la surface intérieure du manchon de montage étant agencée pour coopérer par complémentarité de forme avec la surface extérieure de la partie de raccordement de l'embout de raccordement. La surface extérieure du manchon de montage comporte par exemple un filetage agencé pour coopérer avec le filetage du manchon de verrouillage de l'embout de raccordement.

Selon un autre mode de réalisation de l'invention, la jupe de la portion de protection s'étend le long d'au moins la moitié de la longueur du manchon de verrouillage.

Selon un mode de réalisation de l'invention, le dispositif d'injection est une seringue préremplie.

La seringue préremplie comporte avantageusement :
- un corps tubulaire comprenant une première extrémité ouverte et une deuxième extrémité fermée par une paroi transversale munie d'un orifice d'écoulement, l'embout de raccordement étant disposé au niveau de la deuxième extrémité du corps tubulaire et étant relié fluidiquement à l'orifice d'écoulement,
- un poussoir s'étendant à travers l'ouverture de la première extrémité du corps tubulaire et comportant un piston monté coulissant dans le corps tubulaire, et
- une chambre intérieure délimitée par le corps tubulaire et le piston et contenant un fluide.

De préférence, l'obturateur vient de moulage en une seule pièce avec l'embout de raccordement et le corps tubulaire.

Avantageusement, le corps tubulaire comprend une paroi latérale sensiblement cylindrique. Selon un mode de réalisation de l'invention, la jupe comporte au moins une partie, tournée vers le corps tubulaire, présentant, sur au moins une portion de sa longueur, un diamètre extérieur sensiblement identique à celui de la paroi latérale du corps tubulaire.

Selon un mode de réalisation de l'invention, l'ensemble d'injection comprend un emballage, par exemple de type blister, dans lequel sont conditionné le dispositif d'injection et le dispositif de protection.

De toute façon l'invention sera bien comprise à l'aide de la description qui suit en référence au dessin schématique annexé représentant, à titre d'exemples non limitatifs, plusieurs formes d'exécution de cet ensemble d'injection.
Figures 1 à 5 sont des vues en perspective d'un ensemble d'injection selon un premier mode de réalisation de l'invention dans différentes positions d'utilisation.
Figure 6 est une vue partielle en perspective, à l'échelle agrandie, de l'ensemble d'injection de la figure 1.
Figure 7 est une vue en coupe longitudinale de l'ensemble d'injection de la figure 1.
Figure 8 est une vue partielle en coupe longitudinale, à l'échelle agrandie, de l'ensemble d'injection de la figure 1.
Figure 9 est une vue en coupe longitudinale de l'ensemble d'injection de la figure 1 conditionné dans un emballage.
Figure 10 est une vue partielle en coupe longitudinale d'un ensemble d'injection selon un deuxième mode de réalisation de l'invention.
Figures 11 à 13 sont des vues partielles en perspective d'un ensemble d'injection respectivement selon un troisième, une quatrième et un cinquième modes de réalisation de l'invention.
Figure 14 est une vue en perspective d'un ensemble d'injection selon un sixième mode de réalisation de l'invention.

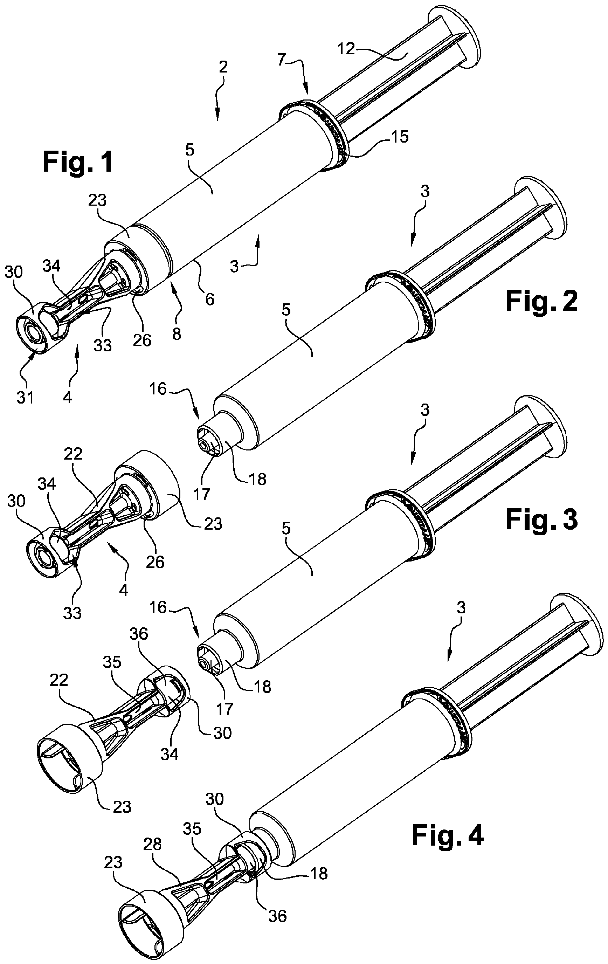

La figure 1 représente un ensemble d'injection 2 selon un premier mode de réalisation de l'invention, qui comprend un dispositif d'injection 3 et un dispositif de protection 4 monté sur le dispositif d'injection 3.

Selon ce premier mode de réalisation de l'invention, le dispositif d'injection 3 est formé par une seringue préremplie et comprend un corps tubulaire 5 comportant une paroi latérale 6 sensiblement cylindrique. La paroi latérale 6 présente une première extrémité 7 ouverte et une deuxième extrémité 8 fermée par une paroi transversale 9 munie en son centre d'un orifice d'écoulement 11.

Le dispositif d'injection 3 comprend également une tige 12 formant poussoir qui s'étend à travers l'ouverture de la première extrémité 7 de la paroi latérale 6. La tige 12 comporte, à son extrémité située dans le corps tubulaire 5, un piston 13 monté coulissant à l'intérieur du corps tubulaire 5 suivant l'axe longitudinal de celui-ci. Le corps tubulaire 5 et le piston 13 délimitent ainsi une chambre intérieure 14 remplie d'un fluide qui peut être par exemple une solution médicamenteuse, un solvant, etc.

La surface externe du corps tubulaire 5 comporte une collerette d'appui 15 au niveau de la première extrémité 7 sur laquelle viennent s'appuyer les doigts de l'utilisateur lorsqu'une poussée est exercée par ce dernier sur l'extrémité de la tige 12 opposée à celle reliée au piston 13.

Le dispositif d'injection 3 comprend en outre un embout de raccordement 16 de type Luer-lock disposé au niveau de la seconde extrémité 8 de la paroi latérale 6 du corps tubulaire 5, sur la face externe de la paroi transversale 9. L'embout de raccordement 16 comprend une partie de raccordement tubulaire 17 communiquant avec l'orifice d'écoulement 11 de la paroi transversale 9, et un manchon de verrouillage 18 disposé coaxialement à la partie de raccordement 17 et entourant cette dernière. La partie de raccordement 17 est avantageusement tronconique, l'ouverture située du côté de plus grand diamètre de la partie de raccordement 17 coïncidant avec l'orifice d'écoulement 11. Le manchon de verrouillage 18 comporte de préférence un filetage 19 ménagé sur sa paroi interne.

Le dispositif d'injection 3 comporte de plus un obturateur 20 relié par une zone sécable 21 à l'extrémité libre de la partie de raccordement 17. Le corps tubulaire 5, l'embout de raccordement 16 et l'obturateur 20 sont par exemple réalisés en matière synthétique et en une seule pièce par moulage.

La zone sécable 21 entre la partie de raccordement 17 et l'obturateur 20 est réalisée avantageusement par un amincissement annulaire de la matière le long de la ligne de raccordement entre l'extrémité libre de la partie de raccordement 17 et l'obturateur 20. Une telle configuration de la zone sécable 21 permet une séparation aisée de l'obturateur 20 et de la partie de raccordement 17.

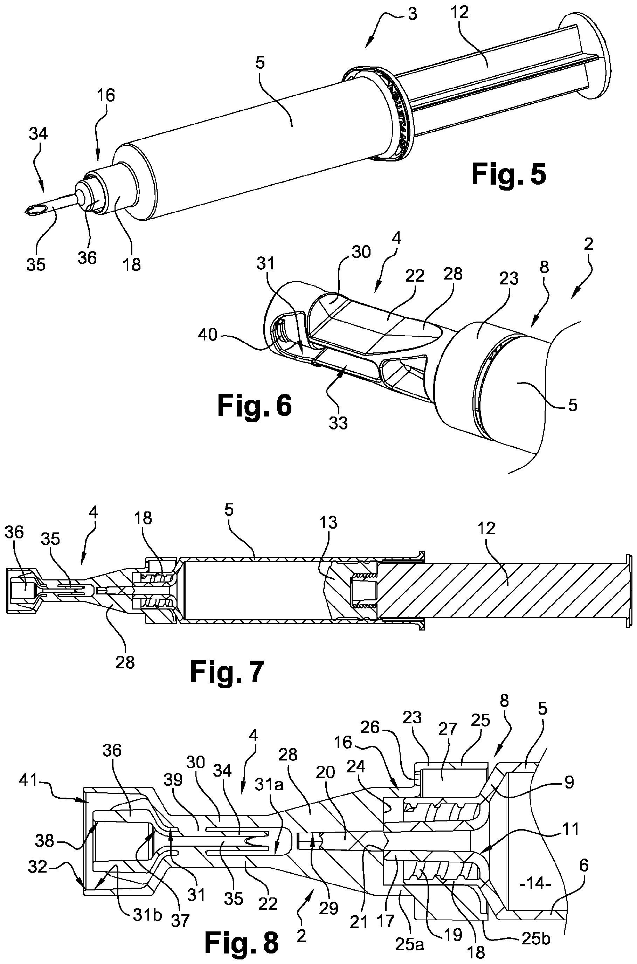

Selon le mode de réalisation représentée sur les figures 6 et 7, le dispositif de protection 4 comprend un corps monobloc 22 comprenant une portion de protection 23 adaptée pour être montée de manière amovible sur l'embout de raccordement 16 du dispositif d'injection 3 de manière préserver la stérilité de l'embout de raccordement 16.

La portion de protection 23 comprend une paroi transversale 24 à partir de laquelle fait saillie une jupe 25 agencée pour venir s'engager sur la paroi externe du manchon de verrouillage 18. La jupe 25 est agencée pour s'étendre au-delà de l'extrémité du manchon de verrouillage 18 tournée vers le corps tubulaire 5 du dispositif d'injection 3, et pour s'étendre de préférence jusqu'à proximité du corps tubulaire 5 du dispositif d'injection 3.

La jupe 25 comprend une première partie cylindrique 25a, et une deuxième partie 25b s'étendant à partir de la première partie 25a. La deuxième partie 25b est tournée vers le corps tubulaire 5 du dispositif d'injection 3, et présente un diamètre extérieur sensiblement identique à celui de la paroi latérale 6 du corps tubulaire 5.

La portion de protection 23 comprend de plus au moins un orifice de passage 26 destiné au passage d'un fluide de stérilisation. L'orifice de passage 26 est par exemple ménagé dans la deuxième partie 25b de la jupe 25. La jupe 25 et le manchon de raccordement 18 sont agencés pour délimiter au moins une chambre interne 27 dans laquelle débouche l'orifice de passage 26. Ainsi, lors de la stérilisation en chaleur humide de l'ensemble d'injection 2, la vapeur peut parvenir depuis l'extérieur du dispositif de protection 4 vers les zones à stériliser de l'embout de raccordement 16. Bien entendu, la jupe 25 pourrait comporter plusieurs orifices de passage 26 débouchant chacun dans une chambre interne 27.

Le corps monobloc 22 comprend également une portion d'accouplement 28 solidaire de la portion de protection 23 et s'étendant à partir de cette dernière. La portion d'accouplement 28 comporte un logement d'accouplement 29 débouchant dans la paroi transversale 24 et dans le volume interne délimité par la jupe 25. Le logement d'accouplement 29 est de préférence centré sur l'axe longitudinal de la jupe 25.

Comme montré sur la figure 7, le logement d'accouplement 29 est agencé pour coopérer avec l'obturateur 20 de sorte que la rotation de la portion d'accouplement 29 autour d'un axe de rotation confondu avec l'axe de la partie de raccordement 17 entraîne en rotation l'obturateur 20 de manière à provoquer la rupture de la zone sécable 21.

Selon le mode de réalisation représenté sur la figure 7, l'obturateur 20 et le logement d'accouplement 29 présentent chacun une forme conique. Cette caractéristique permet l'emmanchement à force de l'obturateur 20 dans le logement d'accouplement 29 de sorte que, après rupture de la zone sécable 21, l'obturateur 20 soit maintenu dans le logement d'accouplement 29 et ne puisse pas tomber au sol. Selon un autre mode de réalisation, l'obturateur 20 et le logement d'accouplement 29 pourrait présenter des formes polygonales complémentaires, par exemple hexagonales.

Le corps monobloc 22 du dispositif de protection 4 comprend également une portion de support 30 solidaire de la portion d'accouplement 28 et s'étendant à partir de cette dernière. La portion de support 30 comprend un logement de stockage 31 comportant une première partie de logement 31a tournée vers la portion d'accouplement 28, et une deuxième partie de logement 31b prolongeant la première partie de logement 31a. La portion de support 30 comprend également une ouverture de passage 32 débouchant dans le logement de stockage 31, et plus particulièrement dans la deuxième partie de logement 31b.

La portion de support 30 comprend de plus deux lumières de passage 33 diamétralement opposée et débouchant chacune dans le logement de stockage 31. Chaque lumière de passage 33 est destinée au passage d'un fluide de stérilisation, et débouche avantageusement dans les première et deuxième parties de logement 31a, 31b.

Le dispositif de protection 4 comprend en outre un élément d'injection 34, de préférence monobloc, monté de manière amovible dans le logement de stockage 31 de la portion de support 30 dans une position de stockage. L'élément d'injection 34 peut être par exemple métallique ou être réalisé en matière plastique.

L'élément d'injection comprend une aiguille d'injection 35 et un embout de connexion 36 relié fluidiquement à l'aiguille d'injection. L'embout de connexion 36 est tubulaire et comporte une première extrémité 37 ouverte reliée fluidiquement à l'aiguille d'injection 35, et une deuxième extrémité 38 ouverte opposée à la première extrémité 37.

L'aiguille d'injection 35 s'étend dans la première partie de logement 31a, et l'embout de connexion 36 s'étend dans la deuxième partie de logement 31b de telle sorte que la deuxième extrémité 38 de l'embout de connexion 36 est située en regard de l'ouverture de passage 32. Il doit bien entendu être noté que l'ouverture de passage 32 est agencée pour permettre le retrait de l'élément d'injection 34 hors du logement de stockage 31. Avantageusement, l'extrémité libre de la portion de support 30 s'étend au-delà de la deuxième extrémité 38 de l'embout de connexion 36.

Selon le mode de réalisation représenté sur les figures 6 et 7, l'élément d'injection 34 est relié à la portion de support 30 par l'intermédiaire d'au moins deux zones ruptibles 39, et vient de moulage en une seule pièce avec le corps monobloc 22. Les zones ruptibles 39 sont plus particulièrement agencées pour relier l'embout de connexion 36 à la portion de support 30.

L'embout de connexion 36 est adapté pour être monté sur l'embout de raccordement 16 du dispositif d'injection 3 dans une position d'utilisation de l'élément d'injection 34. En effet, l'embout de connexion 36 comprend deux ergots de fixation 40 ménagés sur sa paroi extérieure et agencés pour coopérer avec le filetage 19 du manchon de verrouillage 18 de l'embout de raccordement 16. En outre, la paroi intérieure de l'embout de connexion 36 est agencée pour coopérer par complémentarité de forme avec la paroi extérieure de la partie de raccordement 17 de l'embout de raccordement 16.

La portion de support 30 et l'embout de connexion 36 sont agencés de manière à délimiter un espace de passage 41 sensiblement annulaire adapté au passage du manchon de verrouillage 17 de l'embout de raccordement 16 afin de permettre le montage de l'élément d'injection 34 sur l'embout de raccordement 16.

En condition de stockage de l'ensemble d'injection 2, le dispositif d'injection 3 équipé du dispositif de protection 4 sont avantageusement conditionnés dans un blister 50 qui peut être fermé par exemple par un opercule 51 en papier pelable.

Le procédé de raccordement de l'élément d'injection 34 sur le dispositif d'injection 3 va maintenant être décrit en référence plus particulièrement aux figures 1 à 5.

Le procédé de raccordement comprend les étapes suivantes consistant à :
- retirer l'ensemble d'injection 2 du blister dans lequel il est conditionné,
- entraîner en rotation le dispositif de protection 4 autour de son axe longitudinal de manière à sectionner la zone de liaison 21 entre l'obturateur 20 et la partie de raccordement 17,
- retirer le dispositif de protection 4 de manière à libérer l'embout de raccordement 16 (voir la figure 2),
- retourner le dispositif de protection 4 de telle sorte que l'embout de connexion 34 soit tourné vers l'embout de raccordement 16 (voir la figure 3),
- visser l'embout de connexion 36 de l'élément d'injection 34 sur l'embout de raccordement 16 en faisant coopérer les ergots de fixation 40 de l'embout de connexion 36 avec le filetage 19 du manchon de verrouillage 18 (voir la figure 4), et ce jusqu'à sectionner les zones ruptibles 39 reliant l'élément d'injection 34 à la portion de support 30,
- retirer le corps monobloc 22 de l'embout de raccordement 16, l'élément d'injection 34 restant sur le dispositif d'injection 3 du fait de la coopération entre l'embout de connexion 36 et l'embout de raccordement 16 (voir la figure 5).

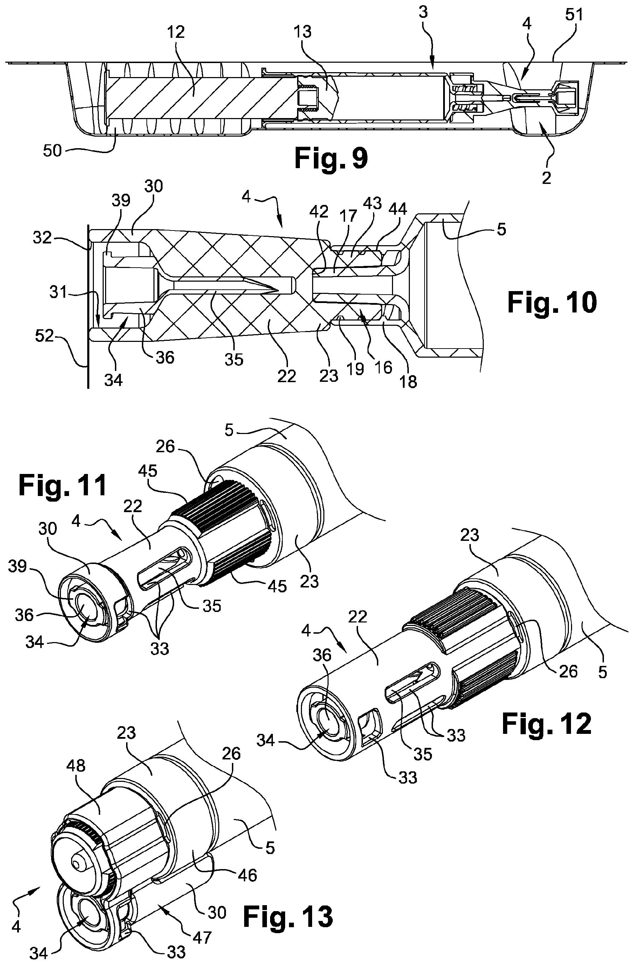

La figure 10 représente un ensemble d'injection 2 selon un deuxième mode de réalisation de l'invention qui diffère de celui représenté sur les figures 1 à 8 essentiellement en ce que le dispositif d'injection 3 est dépourvu d'obturateur et en ce que la portion de protection 23 présente une paroi d'obturation 42 agencée pour obturer l'extrémité libre de la partie de raccordement 17 de l'embout de raccordement 16. Le corps monobloc 22 est alors avantageusement réalisé en élastomère.

De plus, selon ce deuxième mode de réalisation, la portion de protection 23 comporte un manchon de montage 43 présentant une surface extérieure et une surface intérieure. La surface extérieure du manchon de montage 43 comporte un filetage 44 agencé pour coopérer avec le filetage 19 du manchon de verrouillage 18 de l'embout de raccordement 16, tandis que la surface intérieure du manchon de montage 43 est agencée pour coopérer par complémentarité de forme avec la surface extérieure de la partie de raccordement 17 de l'embout de raccordement 16.

En outre, selon ce deuxième mode de réalisation, l'élément d'injection 34 coopère par friction avec une portion de paroi délimitant le logement de stockage 31.

Selon une variante de réalisation, l'ouverture de passage 32 pourrait être obturée par un opercule 52 destiné à préserver la stérilité de l'élément d'injection 34. Un tel opercule devra être retiré par l'utilisateur avant le montage de l'élément d'injection sur l'embout de raccordement 16 afin de préserver la stérilité de ce dernier.

Les figures 11 et 12 représentent un ensemble d'injection 2 selon un troisième et un quatrième modes de réalisation de l'invention qui diffèrent de celui représenté sur les figures 1 à 8 essentiellement en ce que le dispositif de protection 4 présente des cannelures 45 disposées sur la face externe du corps monobloc 22 afin de favoriser sa mise en rotation. Les cannelures 45 sont par exemple ménagées sur la portion d'accouplement 28 du dispositif de protection 4.

Selon ces troisième et quatrième modes de réalisation, chaque lumière de passage 33 débouche soit dans la première partie de logement 31a, soit dans la deuxième partie de logement 31b.

La figure 13 représente un ensemble d'injection 2 selon un cinquième mode de réalisation de l'invention qui diffère de celui représenté sur les figures 1 à 8 essentiellement en ce que le dispositif de protection 4 comprend un capuchon 46 comportant les portions de protection et d'accouplement 23, 28, et un organe de support 47 rapporté sur le capuchon 46 et comportant la portion de support 30 dans laquelle est monté l'élément d'injection 34. L'organe de support 47 comprend avantageusement une portion de montage 48, par exemple annulaire, solidaire de la portion de support 30 et montée sur la portion d'accouplement 28. Selon ce cinquième mode de réalisation, l'ensemble d'injection 34 est décalé par rapport à l'axe longitudinal de l'embout de raccordement 16.

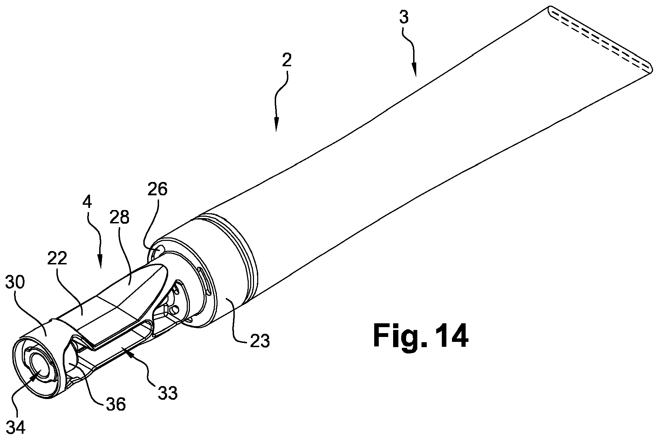

La figure 14 représente un ensemble d'injection 2 selon un sixième mode de réalisation de l'invention qui diffère de celui représenté sur les figures 1 à 8 essentiellement en ce que le dispositif d'injection 3 est un tube d'injection souple.

Comme il va de soi, l'invention ne se limite pas aux seules formes d'exécution de cet ensemble d'injection, décrites ci-dessus à titre d'exemples, elle en embrasse au contraire toutes les variantes de réalisation.

## Revendications

1. Ensemble d'injection (2), comportant :
- un dispositif d'injection prérempli (3) comprenant un embout de raccordement (16), l'embout de raccordement (16) comportant une partie de raccordement tubulaire (17) destinée au passage d'un fluide,
- un obturateur (20) agencé pour obturer l'extrémité libre de la partie de raccordement (17) de l'embout de raccordement (16), l'obturateur (20) étant relié par une zone sécable (21) à l'extrémité libre de la partie de raccordement (17) de l'embout de raccordement (16), et
- un dispositif de protection (4) comprenant :
- un corps monobloc (22) comprenant :
- une portion de protection (23) montée de manière amovible sur l'embout de raccordement (16) du dispositif d'injection (3) de manière à recouvrir et protéger au moins en partie ledit embout de raccordement (16),
- une portion d'accouplement (28) liée à la portion de protection (23) et comportant un logement d'accouplement (29) configuré pour coopérer avec l'obturateur (20), et
- une portion de support (30) liée à la portion d'accouplement (28), et
- un élément d'injection (34) monté de manière amovible sur la portion de support (30) dans une position de stockage, l'élément d'injection (34) comprenant une aiguille d'injection (35) et un embout de connexion (36) relié fluidiquement à l'aiguille d'injection (34), l'embout de connexion (36) étant adapté pour être monté sur l'embout de raccordement (16) du dispositif d'injection (3) dans une position d'utilisation de l'élément d'injection (34), l'élément d'injection (34) et la portion de support (30) étant agencés de telle sorte que, en position de stockage de l'élément d'injection (34), l'embout de connexion (36) est accessible pour coopérer avec l'embout de raccordement (16).

2. Ensemble d'injection selon la revendication 1, dans lequel la portion de support (30) est agencée pour recouvrir au moins en partie l'aiguille d'injection (35) et au moins en partie l'embout de connexion (36).

3. Ensemble d'injection selon la revendication 1 ou 2, dans lequel la portion de support (30) comporte un logement de stockage (31) dans lequel est monté de manière amovible l'élément d'injection (34).

4. Ensemble d'injection selon la revendication 3, dans lequel la portion de support (30) comporte au moins une lumière de passage (33) débouchant dans le logement de stockage (31) et destinée au passage d'un fluide de stérilisation.

5. Ensemble d'injection selon la revendication 3 ou 4, dans lequel la portion de support comporte une ouverture de passage débouchant dans le logement de stockage, l'ouverture de passage étant agencée pour permettre le retrait de l'élément d'injection hors du logement de stockage.

6. Ensemble d'injection selon la revendication 5, dans lequel le logement de stockage comporte une première partie dans laquelle s'étend l'aiguille d'injection, et une deuxième partie prolongeant la première partie et dans laquelle s'étend l'embout de connexion, l'ouverture de passage débouchant dans la deuxième partie du logement de stockage.

7. Ensemble d'injection selon l'une des revendications 1 à 6, dans lequel l'embout de connexion est tubulaire et comporte une première extrémité ouverte reliée fluidiquement à l'aiguille d'injection, et une deuxième extrémité ouverte opposée à la première extrémité ouverte.

8. Ensemble d'injection selon la revendication 5 et l'une des revendications 6 et 7, dans lequel l'ouverture de passage est située en regard de la deuxième extrémité ouverte de l'embout de connexion.

9. Ensemble d'injection selon l'une des revendications 1 à 8, dans lequel l'élément d'injection (34) est relié à la portion de support (30) par l'intermédiaire d'au moins une zone ruptible (39).

10. Ensemble d'injection selon l'une quelconque des revendications 1 à 9, dans lequel la portion d'accouplement (28) est agencée pour coopérer avec l'obturateur (20) de sorte que la rotation de la portion d'accouplement (20) autour d'un axe de rotation parallèle à la direction d'extension de la partie de raccordement (17) entraîne en rotation l'obturateur (20) de manière à provoquer la rupture de la zone sécable (21).

11. Ensemble d'injection selon l'une des revendications 1 à 10, dans lequel l'embout de raccordement (16) du dispositif d'injection (3) est de type Luer ou Luer Lock.

## Patentansprüche

1. Einspritzanordnung (2), umfassend:
- eine vorgefüllte Einspritzvorrichtung (3), die einen Anschlussstutzen (16) einschließt, wobei der Anschlussstutzen (16) einen rohrförmigen Anschlussteil (17) umfasst, der für den Durchgang eines Fluids bestimmt ist,
- einen Verschluss (20), der angeordnet ist, um das freie Ende des Anschlussteils (17) des Anschlussstutzens (16) zu verschließen, wobei der Verschluss (20) über einen teilbaren Bereich (21) mit dem freien Ende des Anschlussteils (17) des Anschlussstutzens (16) verbunden ist, und
- eine Schutzvorrichtung (4), einschließlich:
- eines einstückigen Körpers (22), einschließlich:
- eines Schutzabschnitts (23), der abnehmbar auf dem Anschlussstutzen (16) der Einspritzvorrichtung (3) angebracht ist, sodass der Anschlussstutzen (16) mindestens teilweise bedeckt und geschützt wird,
- eines Kopplungsabschnitts (28), der mit dem Schutzabschnitt (23) verbunden ist und eine Kopplungsaufnahme (29) umfasst, die ausgestaltet ist, mit dem Verschluss (20) zusammenzuwirken, und
- eines Halterungsabschnitts (30), der mit dem Kopplungsabschnitt (28) verbunden ist, und
- eines Einspritzelements (34), das abnehmbar auf dem Halterungsabschnitt (30) in einer Aufbewahrungsposition angebracht ist, wobei das Einspritzelement (34) eine Einspritznadel (35) und einen Verbindungsstutzen (36) einschließt, der fluidisch mit der Einspritznadel (34) verbunden ist, wobei der Verbindungsstutzen (36) angepasst ist, um auf dem Anschlussstutzen (16) der Einspritzvorrichtung (3) in einer Gebrauchsposition des Einspritzelements (34) angebracht zu werden, wobei das Einspritzelement (34) und der Halterungsabschnitt (30) so angeordnet sind, dass in der Aufbewahrungsposition des Einspritzelements (34) der Verbindungsstutzen (36) zugänglich ist, um mit dem Anschlussstutzen (16) zusammenzuwirken.

2. Einspritzanordnung nach Anspruch 1, wobei der Halterungsabschnitt (30) angeordnet ist, um die Einspritznadel (35) mindestens teilweise und den Verbindungsstutzen (36) mindestens teilweise zu bedecken.

3. Einspritzanordnung nach Anspruch 1 oder 2, wobei der Halterungsabschnitt (30) eine Aufbewahrungsaufnahme (31) umfasst, in der das Einspritzelement (34) abnehmbar angebracht ist.

4. Einspritzanordnung nach Anspruch 3, wobei der Halterungsabschnitt (30) mindestens ein Durchgangsloch (33) umfasst, das in die Aufbewahrungsaufnahme (31) mündet und für den Durchgang eines Sterilisationsfluids bestimmt ist.

5. Einspritzanordnung nach Anspruch 3 oder 4, wobei der Halterungsabschnitt eine Durchgangsöffnung umfasst, die in die Aufbewahrungsaufnahme mündet, wobei die Durchgangsöffnung angeordnet ist, um den Rückzug des Einspritzelements aus der Aufbewahrungsaufnahme zu ermöglichen.

6. Einspritzanordnung nach Anspruch 5, wobei die Aufbewahrungsaufnahme einen ersten Teil umfasst, in dem sich die Einspritznadel erstreckt, und einen zweiten Teil, der den ersten Teil verlängert und in dem sich der Verbindungsstutzen erstreckt, wobei die Durchgangsöffnung in den zweiten Teil der Aufbewahrungsaufnahme mündet.

7. Einspritzanordnung nach einem der Ansprüche 1 bis 6, wobei der Verbindungsstutzen rohrförmig ist und ein erstes offenes Ende umfasst, das fluidisch mit der Einspritznadel verbunden ist, und ein zweites offenes Ende, das dem ersten offenen Ende gegenüberliegt.

8. Einspritzanordnung nach Anspruch 5 und einem der Ansprüche 6 und 7, wobei sich die Durchgangsöffnung gegenüber dem zweiten offenen Ende des Verbindungsstutzens befindet.

9. Einspritzanordnung nach einem der Ansprüche 1 bis 8, wobei das Einspritzelement (34) mit dem Halterungsabschnitt (30) über mindestens einen Bruchbereich (39) verbunden ist.

10. Einspritzanordnung nach einem der Ansprüche 1 bis 9, wobei der Kopplungsabschnitt (28) angeordnet ist, um mit dem Verschluss (20) so zusammenzuwirken, dass die Drehung des Kopplungsabschnitts (20) um eine Drehachse parallel zur Ausdehnungsrichtung des Anschlussteils (17) den Verschluss (20) in Drehung versetzt, sodass der Bruch des teilbaren Bereichs (21) ausgelöst wird.

11. Einspritzanordnung nach einem der Ansprüche 1 bis 10, wobei der Anschlussstutzen (16) der Einspritzvorrichtung (3) vom Typ Luer oder Luer-Lock ist.

## Claims

1. An injection assembly (2), including:
- a prefilled injection device (3) comprising a coupling tip (16), the coupling tip (16) including a tubular coupling part (17) intended for the passage of a fluid,
- an obturator (20) arranged for obturating the free end of the coupling part (17) of the coupling tip (16), the obturator (20) being connected by a breakable area (21) to the free end of the coupling part (17), and
- a protection device (4) comprising:
- a one-piece body (22) including:
- a protective portion (23) removably mounted on the coupling tip (16) of the injection device (3) so as to cover and protect at least partially said coupling tip (16),
- a coupling portion (28) connected to the protective portion and including a coupling housing (29) configured to cooperate with the obturator (20), and
- a supporting portion (30) connected to the coupling portion (28), and
- an injecting element (34) removably mounted on the supporting portion (30) in a storage position, the injecting element (34) comprising an injection needle (35) and a connecting tip (36) fluidly connected to the injection needle (35), the connecting tip (36) being adapted to be mounted on the coupling tip (16) of the injection device (3) in a position of use of the injecting element (34), the injecting element (34) and the supporting portion (30) being arranged in such a way that, in the storage position of the injecting element (34), the connecting tip (36) is accessible to cooperate with the coupling tip (16).

2. The injection assembly according to claim 1, wherein the supporting portion (30) is arranged to cover at least partially the injection needle (35) and at least partially the connecting tip (36).

3. The injection assembly according to claim 1 or 2, wherein the supporting portion (30) includes a storage housing (31) in which the injecting element (34) is removably mounted.

4. The injection assembly according to claim 3, wherein the supporting portion (30) includes at least one passage slot (33) opening into the storage housing (31) and intended for the passage of a sterilizing fluid.

5. The injection assembly according to claim 3 or 4, wherein the supporting portion includes a passage aperture opening into the storage housing, the passage aperture being arranged to allow the removal of the injecting element from the storage housing.

6. The injection assembly according to claim 5, wherein the storage housing includes a first part in which the injection needle extends, and a second part forming a continuation of the first part and in which the connecting tip extends, the passage aperture opening into the second part of the storage housing.

7. The injection assembly according to any of claims 1 to 6, wherein the connecting tip is tubular and includes a first open end fluidly connected to the injection needle, and a second open end opposite to the first open end.

8. The injection assembly according to claim 5 and to any of claims 6 and 7, wherein the passage aperture is located facing the second open end of the connecting tip.

9. The injection assembly according to any of claims 1 to 8, wherein the injecting element (34) is connected to the supporting portion (30) via at least one breakable area (39).

10. The injection assembly according to any of claims 1 to 9, wherein the coupling portion (28) is arranged to cooperate with the obturator (20) so that the rotation of the coupling portion (28) about an axis of rotation parallel to the direction of extension of the coupling part (17) drives in rotation the obturator (20) so as to cause the rupture of the breakable area (21).

11. The injection assembly according to any of claims 1 to 10, wherein the coupling tip (16) of the injection device (3) is of the Luer or Luer Lock type.
